(19) Europäisches Patentamt
European Patent Office
Office européen des brevets

(11) **EP 4 111 979 A1**

(12) **EUROPEAN PATENT APPLICATION**

(43) Date of publication:
**04.01.2023 Bulletin 2023/01**

(21) Application number: **22181396.7**

(22) Date of filing: **27.06.2022**

(51) International Patent Classification (IPC):
***A61B 6/00*** *(2006.01)*

(52) Cooperative Patent Classification (CPC):
**A61B 6/566; A61B 6/56;** A61B 6/563; H04W 84/00

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.06.2021 JP 2021106534**

(71) Applicant: **Canon Kabushiki Kaisha**
**Ohta-ku**
**Tokyo 146-8501 (JP)**

(72) Inventors:
• **KOSUGE, Asato**
  **Tokyo, 146-8501 (JP)**
• **MORITA, Hideaki**
  **Tokyo, 146-8501 (JP)**
• **NORO, Toshitaka**
  **Tokyo, 146-8501 (JP)**

(74) Representative: **Canon Europe Limited**
**European Intellectual Property Group**
**4 Roundwood Avenue**
**Stockley Park**
**Uxbridge UB11 1AF (GB)**

(54) **RADIATION IMAGING SYSTEM, RADIATION IMAGING APPARATUS, CONTROL APPARATUS, CONTROL METHOD, AND PROGRAM**

(57)     A radiation imaging system includes a communication device configured to perform wireless communication with a radiation imaging apparatus configured to capture a radiographic image, and an information processing apparatus. The information processing apparatus causes the communication device to perform the wireless communication with the radiation imaging apparatus, based on information about a distance between the communication device and the radiation imaging apparatus and a threshold value set in advance.

## FIG.1

**Description**

BACKGROUND OF THE INVENTION

Field of the Invention

The present invention relates to a radiation imaging system, a radiation imaging apparatus, a control apparatus, a control method, and a program.

Description of the Related Art

A radiation imaging system using radiation is known in the medical field. With digitization of the radiation imaging system, a system is in widespread use, in which a radiation generation apparatus irradiates a radiation imaging apparatus with radiation through a subject, the radiation imaging apparatus captures and generates a digital radiographic image, and an imaging control apparatus enables the image to be viewed immediately after the radiation imaging. This system makes it possible to improve a workflow and capture an image in a faster cycle compared with a conventional imaging method using a film.

As apparatuses in such a radiation imaging system, a radiation imaging apparatus and an imaging control apparatus that are wirelessly connected to eliminate installation restrictions due to a cable of the radiation imaging apparatus are discussed. To establish the wireless connection between the apparatuses, it is necessary to use the same settings, such as a service set identifier (SSID), an authentication method, an encryption type, and an encryption key, between the apparatuses to be connected. Normally, these settings are made manually on both of the apparatuses to be wirelessly connected, or are made using a push button method or a personal identification number (PIN) code method defined by Wi-Fi Protected Setup@.

However, in a case where the settings are manually made, an input operation is required, and there is a possibility that the connection cannot be established due to an error in the operation. Similarly, the PIN code method requires an operation of inputting a PIN code to the master apparatus. Furthermore, the push button method requires an operation that is different from an operation in a normal workflow of the radiation imaging system, such as pressing or touching push buttons of both the slave apparatus and the master apparatus at the same time.

Japanese Patent Application Laid-Open No. 2011-120885 discusses a radiation imaging system including a unit that configures wireless settings between a radiation imaging apparatus and an imaging control apparatus via a connection unit that uses short-range wireless communication, such as infrared communication or Bluetooth®, of which communication range is smaller than that of wireless communication. This radiation imaging system eliminates the need for an operator to manually configure the wireless settings.

However, with the method discussed in Japanese Patent Application Laid-Open No. 2011-120885, in a case where another radiation imaging system is present nearby, there is a possibility that settings for wireless communication with an unintended wireless apparatus of the other radiation imaging system may be made on the radiation imaging apparatus.

SUMMARY OF THE INVENTION

The present invention is directed to providing a radiation imaging system capable of making settings, on a radiation imaging apparatus, for wireless communication with an intended wireless apparatus of the radiation imaging system more easily and more accurately.

According to a first aspect of the present invention, there is provided a radiation imaging system as specified in claims 1 to 14. According to a second aspect of the present invention, there is provided a radiation imaging apparatus as specified in claim 15. According to a third aspect of the present invention, there is provided a control apparatus as specified in claim 16. According to a fourth aspect of the present invention, there is provided a control method as specified in claim 17. According to a fifth aspect of the present invention, there is provided a program as specified in claim 18.

Further features of the present invention will become apparent from the following description of embodiments with reference to the attached drawings.

A radiation imaging system provided according to an aspect of the present invention comprises a communication device configured to perform wireless communication with a radiation imaging apparatus configured to capture a radiographic image, and an information processing apparatus.

The information processing apparatus is configured to cause the communication device to perform the wireless communication with the radiation imaging apparatus, based on information about a distance between the communication device and the radiation imaging apparatus and a threshold value set in advance.

The information processing apparatus may cause the communication device to perform the wireless communication with the radiation imaging apparatus in a case where the information about the distance exceeds the threshold value, and does not cause the communication device to perform the wireless communication with the radiation imaging apparatus in a case where the information about the distance does not exceed the threshold value.

The radiation imaging apparatus may include first wireless communication means for performing wireless communication, different from the wireless communication, with an access point included in the radiation imaging system in order to control the capturing of the image.

The radiation imaging apparatus may include second wireless communication means for performing the wireless communication in order to make a setting to be used for the different wireless communication.

The first wireless communication means may perform the different wireless communication with the access point via a wireless local area network (WLAN).

The second wireless communication means may perform the wireless communication with the communication device via a wireless personal area network (WPAN).

The communication device and the second wireless communication means may support at least one of a Bluetooth@ Basic Rate/Enhanced Data Rate standard and a Bluetooth@ Low Energy standard.

The information about the distance may be based on signal strength of a signal from the communication device acquired by the radiation imaging apparatus.

The information about the distance may be the signal strength corresponding to the distance between the communication device and the radiation imaging apparatus.

In a case where the signal strength exceeds the threshold value, the information processing apparatus may cause the communication device to perform the wireless communication with the radiation imaging apparatus.

The signal strength may be acquired by the second wireless communication means.

The threshold value may be set with respect to the signal strength in a case where the distance is one meter or less.

The information processing apparatus may execute at least one of a plurality of modes for making a response in a case where the communication device is requested by the radiation imaging apparatus to perform the wireless communication.

In an automatic mode among the plurality of modes, a response may be made based on the distance and the threshold value.

In a manual mode among the plurality of modes, a response may be made based on an input using input means included in the information processing apparatus.

In a semi-manual mode among the plurality of modes, a response may be made based on the response in the automatic mode and the response in the manual mode.

In the semi-manual mode, the response may be made based on at least one of a logical sum and a logical product of the response in the automatic mode and the response in the manual mode.

The information processing apparatus may be engaged with and connected to the communication device.

The radiation imaging system may have a plurality of communication devices each configured to perform the wireless communication with the radiation imaging apparatus.

The radiation imaging system may have a plurality of information processing apparatuses each engaged with and connected to a corresponding one of the plurality of communication devices.

A radiation imaging apparatus provided according to an aspect of the present invention comprises wireless communication means for performing wireless communication with a communication device included in a radiation imaging system.

The wireless communication means may start the wireless communication based on information about a distance between the radiation imaging apparatus and the communication device and a threshold value set in advance in the radiation imaging system.

A control apparatus provided according to an aspect of the present invention is configured to, based on information about a distance between a communication device configured to perform wireless communication with the radiation imaging apparatus and the radiation imaging apparatus and a threshold value set in advance, cause the communication device to perform the wireless communication with the radiation imaging apparatus.

A method for controlling a radiation imaging system provided according to an aspect of the present invention comprises a communication device configured to perform wireless communication with a radiation imaging apparatus configured to capture a radiographic image, and an information processing apparatus, the method comprising: comparing information about a distance between the communication device and the radiation imaging apparatus with a threshold value set in advance; and performing the wireless communication based on a result of the comparing.

A computer program provided according to an aspect of the present invention causes a computer to perform processing of the method.

BRIEF DESCRIPTION OF THE DRAWINGS

Fig. 1 is diagram illustrating an example of a configuration of a radiation imaging system according to a first embodiment.
Fig. 2 is a diagram illustrating an example of a configuration of a radiation imaging apparatus according to the first embodiment.
Fig. 3 is a flowchart illustrating an operation performed by the radiation imaging system according to the first embodiment.
Fig. 4 is a sequence diagram illustrating an operation for determining a connection destination according to the first embodiment.
Fig. 5 is a diagram illustrating an example of arrangement of apparatuses according to the first embodiment.
Fig. 6 is a sequence diagram illustrating an operation for determining a connection destination according to a second embodiment.

DESCRIPTION OF THE EMBODIMENTS

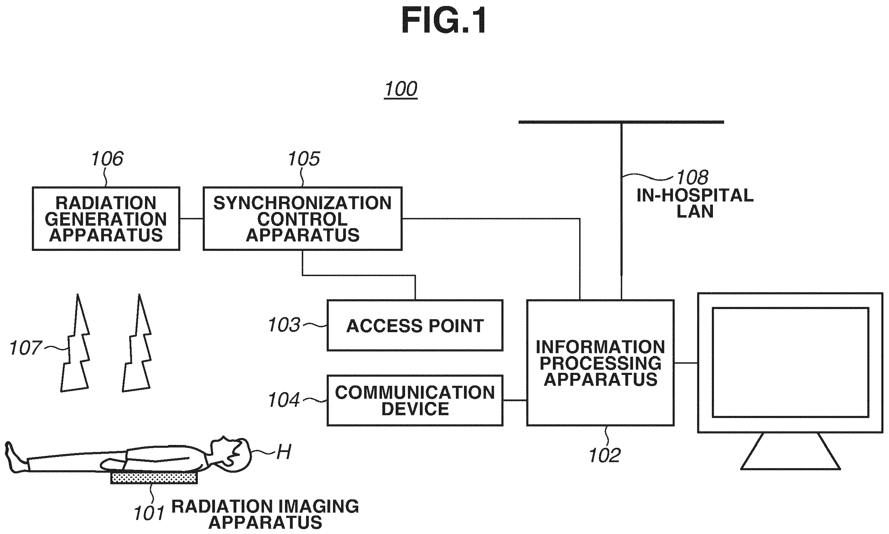

Each of the embodiments of the present invention described below can be implemented solely or as a combination of a plurality of the embodiments or features thereof where necessary or where the combination of elements or features from individual embodiments in a single embodiment is beneficial. A first embodiment of the present invention will be described in detail with reference to Figs. 1 to 5. First, an example of a configuration of a radiation imaging system 100 according to the present embodiment will be described with reference to a schematic diagram of Fig. 1.

The radiation imaging system 100 includes a radiation imaging apparatus 101, an information processing apparatus 102, an access point 103, a communication device 104, a synchronization control apparatus 105, and a radiation generation apparatus 106.

The radiation imaging apparatus 101 captures a radiographic image based on radiation 107 transmitted through a subject H. For example, a portable radiation imaging apparatus is used as the radiation imaging apparatus 101.

The information processing apparatus 102 is implemented by a known technique such as a general-purpose computer, and includes a display unit, an input unit, and a control unit. For example, the information processing apparatus 102 displays, on the display unit, a radiographic image captured by the radiation imaging apparatus 101, or instructs an operator to capture an image via the display unit. The input unit of the information processing apparatus 102 has a function of enabling the operator to input an instruction about imaging conditions and the like. The control unit of the information processing apparatus 102 has a function of comparing acquired signal strength with a threshold value, a function of responding to a connection request, and a function of transmitting setting information about a wireless communication unit.

The access point 103 is a device that relays a radio wave for wirelessly exchanging information between the radiation imaging apparatus 101 and the information processing apparatus 102.

The communication device 104 is connected to the information processing apparatus 102, and transmits and receives a radio wave for performing wireless short-range communication between the radiation imaging apparatus 101 and the information processing apparatus 102. For example, the communication device 104 is a dongle to be connected to the information processing apparatus 102 by a Universal Serial Bus (USB) interface. The communication device 104 supports at least one of a Bluetooth@ Basic Rate/Enhanced Data Rate standard and a Bluetooth@ Low Energy standard.

The communication device 104 may be a radio frequency identifier device (RFID) that exchanges information with a tag in which identification (ID) information is embedded, via short-range wireless communication using an electromagnetic field, a radio wave, or the like. A communication method of the RFID may be an electromagnetic induction method or a radio wave method. The communication device 104 may have an access point function.

While the example in which the communication device 104 is connected to the information processing apparatus 102 has been described above with reference to Fig. 1, the present embodiment is not limited thereto. The communication device 104 may be connected to another apparatus in the radiation imaging system 100, such as the radiation generation apparatus 106. A device incorporated in advance into the radiation imaging system 100 may be used in place of the communication device 104.

The synchronization control apparatus 105 includes a circuit that mediates communication, and monitors states of the radiation imaging apparatus 101 and the radiation generation apparatus 106. For example, the synchronization control apparatus 105 controls the irradiation of the radiation 107 from the radiation generation apparatus 106, and controls the imaging of the subject H by the radiation imaging apparatus 101. The synchronization control apparatus 105 may also include a built-in hub for connecting a plurality of network devices.

The radiation generation apparatus 106 includes, for example, a radiation tube that accelerates electrons at a high voltage and causes the electrons to hit an anode in order to generate the radiation 107 such as X rays. X rays are typically used as the radiation 107. Alternatively, $\alpha$ rays, $\beta$ rays, y rays, or neutron rays may be used as the radiation 107.

An in-hospital local area network (LAN) 108 is established in a hospital and has a function of transmitting and receiving radiographic images captured by the radiation imaging system 100 to and from various places in the hospital.

In the radiation imaging system 100 illustrated in Fig. 1, the radiation generation apparatus 106 irradiates the subject H, which is a patient, with the radiation 107 emitted therefrom. The radiation imaging apparatus 101 generates the radiographic image based on the radiation 107 transmitted through the subject H.

The radiation imaging system 100 can perform imaging using synchronous imaging or asynchronous imaging. The synchronous imaging is an imaging method of matching radiation irradiation timing and imaging timing by exchanging electrical synchronization signals between the radiation imaging apparatus 101 and the radiation generation apparatus 106 via the synchronization control apparatus 105.

The asynchronous imaging is an imaging method in which the radiation imaging apparatus 101 starts imaging upon detection of incidence of radiation without exchanging the electrical synchronization signals between the radiation imaging apparatus 101 and the radiation generation apparatus 106. In the case of the asynchronous imaging, the radiation imaging apparatus 101 may transfer a captured radiographic image in each imaging or store the captured radiographic image therein without transferring the image in each imaging.

The radiation imaging system 100 can perform imaging with imaging conditions generally used in radiation imaging, such as fluoroscopic imaging, continuous imaging, still picture imaging, digital subtraction angiography (DSA) imaging, road map imaging, program imaging, tomography imaging, or tomosynthesis imaging.

On the radiation imaging system 100, various functional settings are made, such as an imaging frame rate, a tube voltage, a tube current, a sensor readout area, a sensor drive binning setting, a collimator aperture setting, a radiation window width, and whether to accumulate captured radiographic images in the radiation imaging apparatus 101. In addition, functional settings such as an auto dose control (ADC) setting and an auto exposure control (AEC) setting may be made on the radiation imaging system 100.

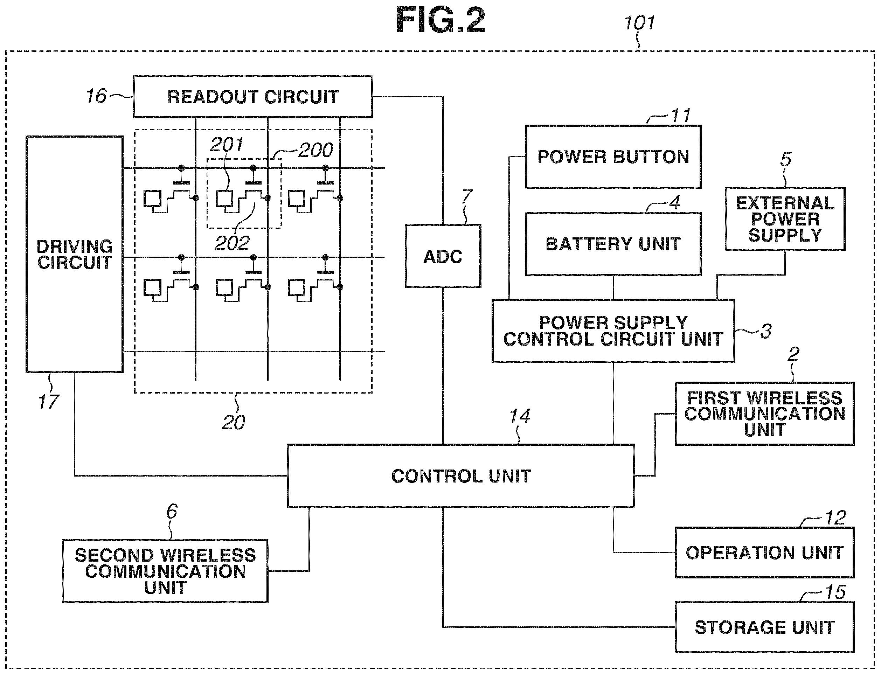

Next, an example of a configuration of the radiation imaging apparatus 101 will be described with reference to Fig. 2. A power button 11 is an operation unit for issuing an instruction to start or stop power supply to each component in the radiation imaging apparatus 101. Examples of the operation unit include a device having this function, a circuit having this function, and a circuit described by a program. The operator operates the power button 11 to prepare for imaging. The power button 11 is provided, for example, on a side surface of the radiation imaging apparatus 101, but may be provided on any surface other than a radiation incident surface.

A battery unit 4 supplies a predetermined voltage to each component in the radiation imaging apparatus 101. For example, a lithium-ion battery or an electric double layer capacitor is used for the battery unit 4. In a case where power is constantly supplied to the radiation imaging apparatus 101 from an external power supply 5, the battery unit 4 may not necessarily be provided.

The external power supply 5 supplies a predetermined voltage to each component in the radiation imaging apparatus 101 from the outside of the radiation imaging apparatus 101. The external power supply 5 may supply power in a wired manner or in a noncontact manner.

Depending on an operating state of the power button 11, a power supply control circuit unit 3 controls the power supply from the battery unit 4 or the external power supply 5 to each component in the radiation imaging apparatus 101, and monitors a battery remaining amount of the battery unit 4. For example, the power supply control circuit unit 3 transforms the voltage from the battery unit 4 or the external power supply 5 into a predetermined voltage and supplies the voltage to each component in the radiation imaging apparatus 101. Furthermore, for example, in a case where the external power supply 5 is not connected to the radiation imaging apparatus 101, the power supply control circuit unit 3 switches between execution and non-execution of the power supply from the battery unit 4 in response to an operation on the power button 11.

A radiation detection unit 20 detects the radiation 107 transmitted through the subject H, as image signals (electric charges). The radiation 107 transmitted through the subject H is incident on a fluorescent substance included in the radiation detection unit 20 and is converted into light by the fluorescent substance. The light is converted into image signals (electric charges) by photoelectric conversion elements 201 of a plurality of pixels 200 arranged in a two-dimensional array. The image signals (electric charges) are read out by a readout circuit 16 and a driving circuit 17.

When the pixels 200 in a certain row are selected by a drive signal generated by the driving circuit 17, switch elements 202 of the pixels 200 in the row are sequentially turned on. The image signals (electric charges) accumulated in the photoelectric conversion elements 201 of the pixels 200 in the row are output to a signal line connected to the pixels 200. The readout circuit 16 has a function of amplifying the image signals (electric charges) output to the signal line and sequentially reads out the image signals from the radiation detection unit 20.

An analog-to-digital converter (ADC) 7 converts the analog image signals read out by the readout circuit 16 into digital image signals and outputs the digital image signals to a control unit 14, as a radiographic image. In other words, the ADC 7 is an A/D conversion unit that converts the analog image signals read out by the readout circuit 16 into digital data.

A storage unit 15 stores the radiographic image data output from the ADC 7, a system identifier, a threshold value of radio wave strength between the radiation imaging apparatus 101 and the communication device 104, and an offset image. The storage unit 15 may also store a technician ID that is identification information about a technician corresponding to the generated image data, a patient ID that is identification information about a patient corresponding to the generated image data, the imaging conditions including an imaging time, an imaging dose, an imaging region, and the number of captured images, and a transfer history of the radiographic image data.

The storage unit 15 is a readable and writable device and, more specifically, a nonvolatile memory such as a flash memory. However, the storage unit 15 is not limited thereto and may be a volatile storage device such as a synchronous dynamic random access memory (SDRAM). The storage unit 15 may be a detachable device such as a secure digital (SD) card and may be configured to be attached to the information processing apparatus 102.

A first wireless communication unit 2 communicates with the access point 103 via a wireless LAN (WLAN) to transmit and receive data such as a radiographic image to and from the information processing apparatus 102, and to control the radiation imaging by the radiation imaging apparatus 101 from the information processing apparatus 102. A wireless communication module is set in the first wireless communication unit 2 based on a medium to be used for communication with the information processing apparatus 102 or the synchronization control apparatus 105. For the first wireless communication unit 2, the control unit 14 can set transmission power of a wireless radio wave.

The first wireless communication unit 2 stores information such as a data rate, a packet loss amount per unit time, a round trip time (RTT), and a packet buffer occupancy rate. The first wireless communication unit 2 also stores information about a received signal strength indicator (RSSI) and a signal-to-noise ratio (SNR) of a signal in wireless communication.

A second wireless communication unit 6 communicates with the communication device 104 via a wireless personal area network (WPAN). As the second wireless communication unit 6, a device that supports at least one of the Bluetooth® Basic Rate/Enhanced Data Rate standard and the Bluetooth@ Low Energy standard is used depending on the communication device 104 to be communicated with. Through communication with the communication device 104, the second wireless communication unit 6 can transmit and receive information such as an identifier of the radiation imaging system 100 and a service set identifier (SSID) and an encryption key that are to be used for communication with the first wireless communication unit 2. For the second wireless communication unit 6, the control unit 14 can set transmission power of a wireless radio wave.

A wireless communication module is set in the second wireless communication unit 6 based on a medium to be used for communication with the information processing apparatus 102 or the synchronization control apparatus 105. The second wireless communication unit 6 stores information such as a data rate, a packet loss amount per unit time, an RTT, and a packet buffer occupancy rate. The second wireless communication unit 6 can also store information about the RSSI and the SNR of a signal in wireless communication.

An operation unit 12 can be used as a manual trigger for transmitting and receiving the setting information between the radiation imaging apparatus 101 and the communication device 104. For example, an operation on the operation unit 12 enables the transmission and reception of the identifier of the radiation imaging system 100, the SSID and the encryption key to be set on the first wireless communication unit 2, and the like. The operation unit 12 is provided, for example, on the side surface of the radiation imaging apparatus 101, but may be provided on any surface other than the radiation incident surface.

The control unit 14 switches whether to enable communication with an external device such as the communication device 104, and controls the communication with the external device. In a case where the second wireless communication unit 6 is controlled to perform wireless communication or in a case where an instruction for acquiring the signal strength is issued through the operation unit 12, the control unit 14 transmits the signal strength in the communication with the communication device 104, via the second wireless communication unit 6. Then, in a case where the transmitted signal strength exceeds the threshold value of the signal strength stored in advance in the information processing apparatus 102, the control unit 14 can start a connection with the communication device 104.

For example, the radiation imaging apparatus 101 transmits advertising packets to broadcast data such as an identifier indicating the radiation imaging apparatus 101 or a device address. The information processing apparatus 102 determines whether the data in the advertising packets received via the communication device 104 matches the identifier or the device address stored in advance. The radiation imaging apparatus 101 can start a connection with the communication device 104 in a case where the signal strength of the received advertising packets is acquired and the acquired signal strength exceeds the threshold value of the signal strength stored in advance in the information processing apparatus 102.

After starting the connection, the control unit 14 communicates the system identifier and the communication settings (the SSID and the encryption key) regarding the first wireless communication unit 2. Subsequently, in a case where there is a difference between the newly set communication settings and the currently set communication settings, the control unit 14 controls the first wireless communication unit 2 to communicate with the access point 103 using the newly set communication settings.

At this time, the newly set communication settings may be stored in the storage unit 15. Furthermore, in a case where the communication is performed using the newly set communication settings, a display may be performed on a display unit (not illustrated) of the radiation imaging apparatus 101. The system identifier may be included in the advertising packets or in a response (SCAN_RESP) packet in making an active scan request (SCAN_REQ) before the connection.

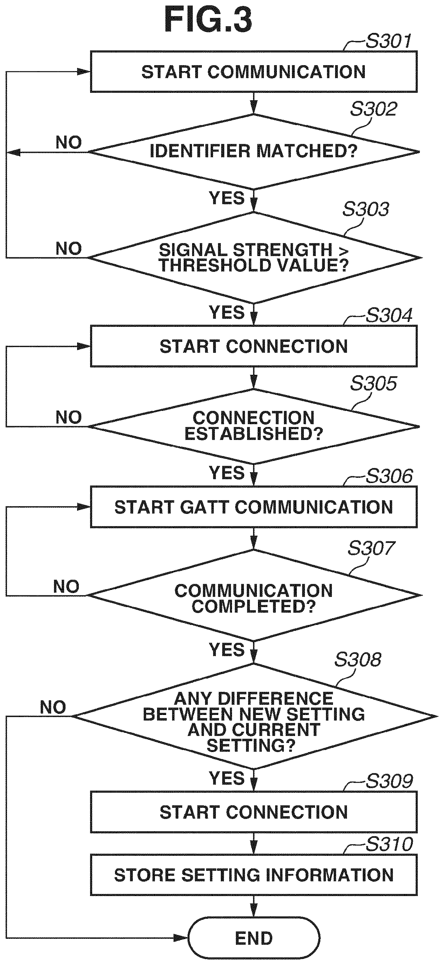

Next, an operation performed by the radiation imaging system 100 before the connection between the first wireless communication unit 2 and the access point 103 is established will be described with reference to a flowchart in Fig. 3.

In step S301, to start communication for exchanging information about the communication settings, the radiation imaging apparatus 101 broadcasts information such as the signal strength at regular intervals, and the information processing apparatus 102 receives the broadcast packets. A trigger for starting the communication may be an operation on the operation unit 12 or control by software in the information processing apparatus 102. Then, the operation proceeds to step S302.

In step S302, in a case where the information in the received advertising packets matches the identifier or the device address stored in advance (YES in step S302), the information processing apparatus 102 acquires the signal strength between the communication device 104 and the radiation imaging apparatus 101, and then the operation proceeds to step S303.

In a case where the information in the received advertising packets does not match the identifier or the device address stored in advance (NO in step S302), the operation returns to step S301.

In step S303, the information processing apparatus 102 determines whether the signal strength of the communication between the communication device 104 and the radiation imaging apparatus 101 exceeds the threshold value set in advance in the information processing apparatus 102. In a case where the information processing apparatus 102 determines that the signal strength exceeds the threshold value (YES in step S303), the operation proceeds to step S304. In a case where the signal strength does not exceed the threshold value (NO in step S303), the operation returns to step S301.

In step S304, the communication device 104 starts a connection with the second wireless communication unit 6. Then the operation proceeds to step S305.

In step S305, in a case where the connection between the communication device 104 and the second wireless communication unit 6 is established (YES in step S305), the operation proceeds to step S306. In a case where the connection is not established (NO in step S305), the operation returns to step S304.

In step S306, the communication device 104 starts data communication using Generic Attribute Profile (GATT) that defines a method for data structuring and a method for exchanging data between applications. For example, the second wireless communication unit 6 and the communication device 104 are set to act as a client and a server, respectively. Then the operation proceeds to step S307.

In step S307, the communication device 104 sets the communication settings (the SSID and the encryption key) according to an appropriate protocol (e.g., a protocol specifying that information to be used should be received in predetermined order), and in a case where the communication is completed (YES in step S307), the operation proceeds to step S308. In a case where the communication fails (NO in step S307), the operation returns to step S306.

A Universal Unique Identifier (UUID) for uniquely identifying a service of the GATT may be defined in advance between the radiation imaging apparatus 101 and the information processing apparatus 102, or negotiations may be performed during the communication.

Furthermore, the communication settings may include setting of a wireless channel, setting of an internet protocol (IP) address of the radiation imaging apparatus 101, setting of the master apparatus and the slave apparatus, setting of an encryption method, and setting of an IP address of the synchronization control apparatus.

The communication device 104 may communicate the system identifier in step S307 instead of step S302.

In step S308, in a case where the communication device 104 determines that there is a difference between the newly set communication settings and the currently set communication settings (YES in step S308), the operation proceeds to step S309. In a case where there is no difference therebetween (NO in step S308), the operation in the flowchart ends.

In step S309, the communication device 104 controls the first wireless communication unit 2 or the second wireless communication unit 6 to perform communication using the communication settings newly set in the steps up to step S309. Then the operation proceeds to step S310.

In step S310, the communication device 104 stores the newly set communication settings in the information processing apparatus 102. This is the end of the flowchart.

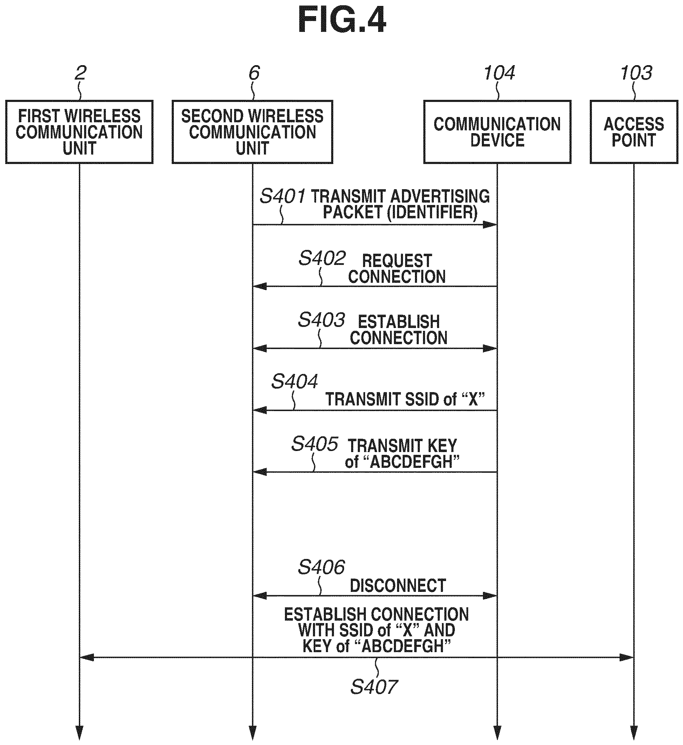

Next, the operation performed by the radiation imaging system 100 before the connection between the first wireless communication unit 2 and the access point 103 is established will be described with reference to a sequence diagram in Fig. 4. Fig. 4 illustrates an operation example in a case where the radiation imaging apparatus 101 is a broadcaster (an advertiser) that broadcasts data and the information processing apparatus 102 is an observer (a scanner) that monitors the data.

In step S401, after startup, the radiation imaging apparatus 101 acts as the advertiser that broadcasts advertising packets in order to establish a connection with any of a plurality of the information processing apparatuses 102. The radiation imaging apparatus 101 transmits the information including the system identifier to the communication device 104 via the second wireless communication unit 6. The transmission power and a packet transmission cycle in the transmission can be set in advance.

At this time, the display unit (not illustrated) of the radiation imaging apparatus 101 may perform a display indicating that the connection with the information processing apparatus 102 is being established. For example, the display unit may be configured with a light emitting diode (LED) and indicate that the connection with the information processing apparatus 102 is being established by blinking the LED.

The communication device 104 acts as the scanner that is ready to receive the advertising packets. In a case where the information processing apparatus 102 recognizes the radiation imaging apparatus 101 as a connection partner based on the system identifier received from the second wireless communication unit 6 by the communication device 104, the information processing apparatus 102 make a determination about the signal strength based on the predetermined threshold value. In a case where the signal strength is determined to exceed the threshold value, the operation proceeds to step S402. In a case where the signal strength is determined to be the threshold value or less, the operation in step S401 is performed again.

When the broadcast of the advertising packets is started, a timeout period is set in the control unit 14 of the radiation imaging apparatus 101. In a case where it is not determined that the identifiers match between the second wireless communication unit 6 and the communication device 104 and the signal strength exceeds the threshold value before the timeout period expires after the start of broadcast of the advertising packets, the radiation imaging apparatus 101 cancels the broadcast.

In a case where the radiation imaging apparatus 101 cancels the broadcast upon timeout, the display unit of the radiation imaging apparatus 101 may perform a display indicating that the broadcast of the advertising packets has been canceled. For example, the display unit may be configured with an LED and indicate that the broadcast of the advertising packets has been canceled by turning off the blinking LED or blinking the LED at a different interval.

In addition, the connection may be canceled by an operation on the power button 11 of the radiation imaging apparatus 101 before it is determined that the identifiers match between the second wireless communication unit 6 and the communication device 104 and the signal strength exceeds the threshold value. In this case, similarly to the case of timeout, the display unit may indicate the cancellation of the connection by turning off the LED or blinking the LED at a different interval.

In step S402, the communication device 104 transmits a connection request to the second wireless communication unit 6, and then the operation proceeds to step S403. The connection request may be transmitted by the operator performing an operation using the input unit of the information processing apparatus 102. In a case where the operation in step S402 is performed, the communication device 104 enters an initiating state that is a connection start state.

When the second wireless communication unit 6 receives the connection request from the communication device 104, a link timeout period is set in the control unit 14. In a case where a connection is not established between the communication device 104 and the second wireless communication unit 6 before the link timeout period expires, the radiation imaging apparatus 101 notifies the information processing apparatus 102 that the connection has failed.

Upon receiving the notification, the information processing apparatus 102 cancels the establishment of the connection with the radiation imaging apparatus 101. At this time, the display unit of the radiation imaging apparatus 101 may perform a display indicating that the establishment of the connection has been canceled. Also in this case, similarly to the case of timeout, the display unit may indicate the cancellation of the connection by turning off the blinking LED or blinking the LED at a different interval.

In step S403, the second wireless communication unit 6 and the communication device 104 are in a connected state. At this time, pairing, which is sharing of the encryption key, and a search for the partner's services may be performed between the second wireless communication unit 6 and the communication device 104. Then, the operation proceeds to step S404.

In step S404, the communication device 104 transmits the SSID of the access point 103 to the second wireless communication unit 6. For example, the SSID is a character string of "X". The SSID is desirably encrypted, but may not necessarily be encrypted. Then, the operation proceeds to step S405.

In step S405, the communication device 104 transmits the encryption key of the access point 103 to the second wireless communication unit 6. For example, the encryption key is a character string of "ABCDEFGH". The encryption key is desirably encrypted, but may not necessarily be encrypted. Then the operation proceeds to step S406.

In step S406, the connection between the communication device 104 and the second wireless communication unit 6 is disconnected, and then the operation proceeds to step S407.

In step S407, the control unit 14 of the radiation imaging apparatus 101 controls the first wireless communication unit 2 to connect to the access point 103, using the communication settings received in steps S404 and S405. The access point 103 performs authentication using the new communication settings and performs communication.

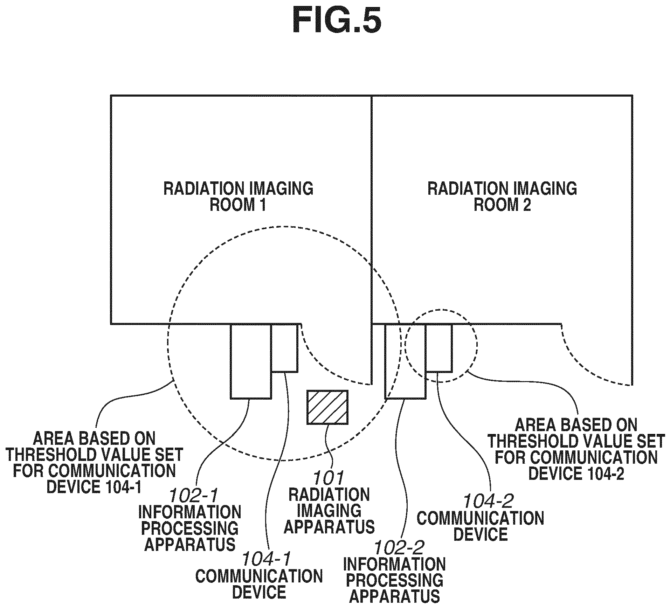

Next, a method for connecting the radiation imaging apparatus 101 and the information processing apparatus 102 in a case where a plurality of the information processing apparatuses 102 is present nearby will be described with reference to an example of arrangement of apparatuses in Fig. 5. In Fig. 5, it is assumed that the radiation imaging apparatus 101 is the broadcaster (the advertiser) that broadcasts data and the information processing apparatus 102 is the observer (the scanner) that monitors the data. It is also assumed in Fig. 5 that an imaging room in which an apparatus is used is distinguished by putting a number after a hyphen in the name of the apparatus. For example, the information processing apparatuses 102-1 and 102-2 are used in radiation imaging rooms 1 and 2, respectively.

Assume a case where a plurality of the communication devices 104 (104-1 and 104-2) is nearby as illustrated in Fig. 5 in transmitting and receiving the setting information as described with reference to Figs. 3 and 4. In a case where the radiation imaging apparatus 101 acts as the advertiser that broadcasts the advertising packets, both of the communication devices 104-1 and 104-2 are determined as connection target devices.

In this case, the SSID and the encryption key associated with the information processing apparatus 102 connected to the communication device 104 that is faster in establishing the connection are set on the radiation imaging apparatus 101. Thus, there is a possibility that the radiation imaging apparatus 101 may be connected to the unintended information processing apparatus 102.

To address the issue, in the present embodiment, the information processing apparatus 102 causes the communication device 104 to wirelessly communicate with the radiation imaging apparatus 101, based on information about a distance between the radiation imaging apparatus 101 and the communication device 104 and a threshold value set in advance for the radiation imaging apparatus 101. In the present embodiment, the threshold value can be set by each of the information processing apparatuses 102. This makes it possible to prevent an unintended combination of the radiation imaging apparatus 101 and the information processing apparatus 102 from being connected to each other.

The information about the distance between the radiation imaging apparatus 101 and the communication device 104 is a value related to the distance between the radiation imaging apparatus 101 and the communication device 104 acquired by some sort of unit. The information about the distance may be a value acquired by a unit such as a sensor capable of measuring the distance between the communication device 104 and the radiation imaging apparatus 101, or may be a value that changes based on the distance between the communication device 104 and the radiation imaging apparatus 101. In the present embodiment, the signal strength of a signal from the communication device 104, which is acquired by the radiation imaging apparatus 101, is used as the information about the distance. A specific method for connecting the radiation imaging apparatus 101 and the information processing apparatus 102 in a case where the signal strength is used as the information about the distance will be described next.

The information processing apparatus 102-1 sets the signal strength corresponding to a distance of one meter as the threshold value so that the connection request is returned if the distance between the communication device 104-1 and the second wireless communication unit 6 of the radiation imaging apparatus 101 is one meter or less. The information processing apparatus 102-2 sets the signal strength corresponding to a distance of 0.1 meters as the threshold value so that the connection request is returned if the distance between the communication device 104-2 and the second wireless communication unit 6 of the radiation imaging apparatus 101 is 0.1 meters or less.

In Fig. 5, two areas are indicated by dotted circles. One is an area in which the communication device 104-1 returns the connection request based on the threshold value set by the information processing apparatus 102-1 for the radiation imaging apparatus 101. The other is an area in which the communication device 104-2 returns the connection request based on the threshold value set by the information processing apparatus 102-2 for the radiation imaging apparatus 101. As illustrated in Fig. 5, these two areas do not overlap each other. Thus, it is possible to prevent an unintended combination of the radiation imaging apparatus 101 and the information processing apparatus 102 from being connected to each other.

Because the relationship between the distance and the signal strength differs depending on the output of a wireless module or the like, it is desirable that the relationship between the distance and the signal strength be measured and the threshold value of the signal strength be set for each use environment.

The signal strength ideally becomes weaker as the distance increases, but in an actual use environment, variation in the value of signal strength increases as the distance increases due to influence of a reflected wave or the like. Thus, the threshold value of the signal strength set by the information processing apparatus 102 is desirably the signal strength in a case where the distance between the communication device 104-1 and the second wireless communication unit 6 is one meter or less.

The relationship between the distance and the signal strength will be described now. Because the signal strength has a characteristic of being attenuated in proportion to the square of the distance, the distance between the devices can be calculated from the signal strength acquired by the second wireless communication unit 6. In a case where received power of a reception antenna is calculated from power radiated from a transmission antenna, the Friis transmission equation is to be used.

For example, the radiation imaging apparatus 101 and the communication device 104 are arranged at positions separated by a distance XX (unit: m), and signal strength YY (unit: dBm) is acquired by the first wireless communication unit 2 or the second wireless communication unit 6 of the radiation imaging apparatus 101. At this time, calibration data CAL can be expressed by the following formula (1).

$$CAL = 20 * \log(XX) + YY \quad (1)$$

Assuming that the signal strength YY at a distance ZZ is $\alpha$, a theoretical value of the distance ZZ can be expressed by the following formula (2).

$$ZZ = 10\^{((CAL - \alpha)/20)} \quad (2)$$

The signal strength may be determined by performing sampling a plurality of times and using an average value, a median value, a maximum value, a minimum value, or a standard deviation of values acquired in the plurality of times of sampling, in consideration of influence of variation in the acquired values.

The calibration data CAL may be acquired during manufacturing in a factory. However, to acquire a more accurate value, it is desirable that the calibration data CAL be acquired in the actual use environment in consideration of the characteristics of a wireless device and the influence of a room environment. The relationship between the distance XX and the signal strength YY in the calibration data CAL is calculated by measuring the signal strength YY at one or more points at the distance XX. It is more desirable that the calibration data CAL be acquired by measuring the signal strength YY at two or more points at the distance XX.

It is desirable that at least one of the distances XX in the calibration data CAL be zero meters in order to correct the signal strength corresponding to the distance between the radiation imaging apparatus 101 and each of the communication devices 104 due to individual variations among the communication devices 104. Furthermore, in a case where the signal strength is acquired, it is desirable to acquire an arbitrary number of values to suppress the influence of multipath and wireless interference by using an arithmetical mean or the like.

As described above, according to the present embodiment, the information processing apparatus 102 causes the communication device 104 and the radiation imaging apparatus 101 to perform wireless communication, based on the information about the distance between the communication device 104 and the radiation imaging apparatus 101 and the threshold value set in advance. The threshold value can be set by the information processing apparatus 102 included in each of a plurality of the radiation imaging systems 100. The threshold value can be set using, for example, the input unit included in the information processing apparatus 102.

This makes it possible to avoid an overlap of the respective areas in which the plurality of the radiation imaging systems 100 performs the wireless communication, and to prevent wireless settings from being configured between the radiation imaging apparatus 101 and the unintended communication device 104.

As the information about the distance between the communication device 104 and the radiation imaging apparatus 101, the signal strength of the signal from the communication device 104, which is acquired by the radiation imaging apparatus 101, can be used. In this case, the information processing apparatus 102 causes the communication device 104 and the radiation imaging apparatus 101 to perform the wireless communication, based on a result of comparing the signal strength of the signal from the communication device 104 acquired by the radiation imaging apparatus 101 with the threshold value set in advance.

More specifically, the information processing apparatus 102 causes the communication device 104 and the second wireless communication unit 6 of the radiation imaging apparatus 101 to perform the wireless communication in a case where the signal strength is the threshold value or more. The second wireless communication unit 6 functions as a reception unit configured to receive the setting information that is transmitted from the communication device 104 and is to be used for the wireless communication between the first wireless communication unit 2 and the access point 103.

In the above description, the signal strength is used as the information about the distance between the communication device 104 and the radiation imaging apparatus 101, but the present embodiment is not limited thereto. For example, a measuring instrument capable of measuring the distance may be additionally provided in the radiation imaging apparatus 101, and a value measured by the instrument may be used.

For example, the radiation imaging apparatus 101 may be provided with an acceleration sensor, a value measured by the acceleration sensor may be integrated to calculate a speed, the speed may be further integrated to calculate a distance, and the calculated value may be used as the information about the distance between the communication device 104 and the radiation imaging apparatus 101. The radiation imaging apparatus 101 may also be provided with a laser length-measurement device, and a value acquired by the laser length-measurement device may be used as the information about the distance between the communication device 104 and the radiation imaging apparatus 101. The information about the distance between the communication device 104 and the radiation imaging apparatus 101 may also be acquired using other known techniques.

In the present embodiment, the information processing apparatus 102 executes a plurality of modes for responding to the connection request from the communication device 104. A mode for automatically responding to the connection request based on the threshold value set in advance for the radiation imaging apparatus 101 and the acquired signal strength is referred to as an automatic mode.

A mode in which the information processing apparatus 102 responds to the connection request based on an input by the operator using the input unit of the information processing apparatus 102 regardless of the threshold value set in advance for the radiation imaging apparatus 101 is referred to as a manual mode. More specifically, in the manual mode, when the information processing apparatus 102 receives the advertising packets from the radiation imaging apparatus 101, the display unit of the information processing apparatus 102 perform a display indicating that the connection request has been received from the radiation imaging apparatus 101 that is the transmission source of the advertising packets. The operator inputs the response to the connection request, using the input unit of the information processing apparatus 102.

A mode for responding to the connection request based on the response in the automatic mode and the response in the manual mode is referred to as a semi-manual mode. In the semi-manual mode, for example, the response to the connection request is made based on a logical sum of the response in the automatic mode and the response in the manual mode. More specifically, the wireless communication between the communication device 104 and the radiation imaging apparatus 101 is started in a case where the response indicating the start of the wireless communication is made based on one of the determination that the signal strength exceeds the threshold value and the response by the input of the operator to the input unit.

Furthermore, in the semi-manual mode, the response may be made based on a logical product of the response in the automatic mode and the response in the manual mode. More specifically, the wireless communication between the communication device 104 and the radiation imaging apparatus 101 may be started in a case where the signal strength exceeds the threshold value and the response is made by the input of the operator to the input unit. In other words, in the semi-manual mode, the response is made based on at least one of the logical sum and the logical product of the response in the automatic mode and the response in the manual mode.

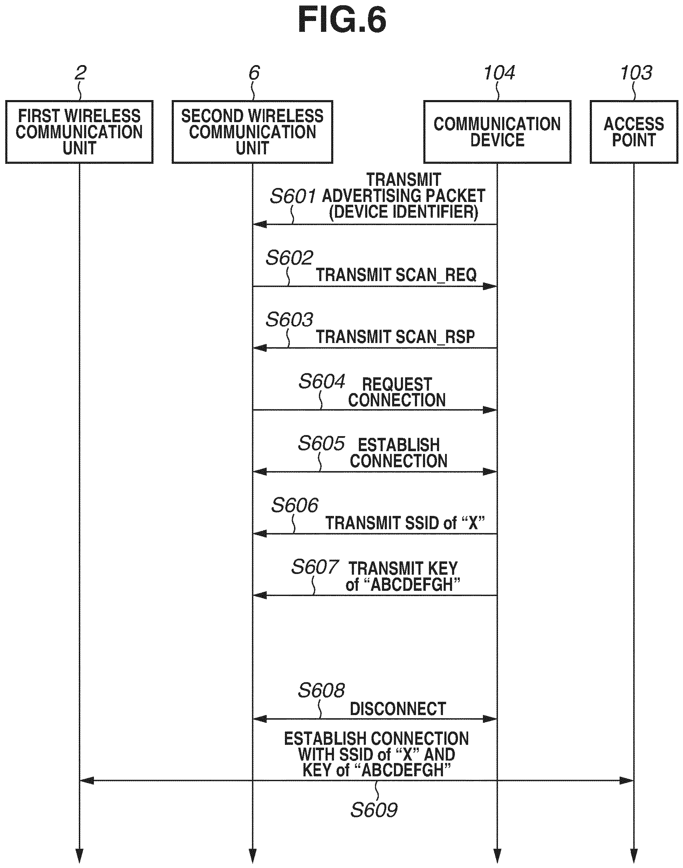

In a second embodiment, the role of the observer and the role of the broadcaster are changed from those in the first embodiment described with reference to Fig. 4. An operation example in a case where the radiation imaging apparatus 101 is the observer (the scanner) that monitors data and the information processing apparatus 102 is the broadcaster (the advertiser) that broadcasts the data will be described with reference to a sequence diagram in Fig. 6. Descriptions of parts similar to those in the first embodiment will be omitted.

In step S601, after startup, the information processing apparatus 102 acts as the advertiser that broadcasts advertising packets and transmits information including the device address to the second wireless communication unit 6 by using the communication device 104. At this time, the transmission power and the packet transmission cycle can be set in advance. The second wireless communication unit 6 acts as the scanner that is ready to receive the advertising packets. In a case where the second wireless communication unit 6 receives the advertising packets and recognizes the information processing apparatus 102 as the connection partner based on the device address, the operation proceeds to step S602.

In step S602, in a case where the data in the advertising packets does not contain sufficient information, the second wireless communication unit 6 transmits a SCAN_REQ, and the operation proceeds to step S603.

In step S603, upon receiving the SCAN_REQ, the communication device 104 transmits a SCAN_RSP packet to the second wireless communication unit 6. The second wireless communication unit 6 receives the SCAN_RSP packet, and the operation proceeds to step S604. In step S604, the second wireless communication unit 6 enters the initiating state that is the connection start state.

The operation in steps S604 to S609 is similar to the operation in steps S402 to S407, respectively.

According to the above-described embodiments of the present invention, it is possible to make settings, on the radiation imaging apparatus, for wireless communication with the intended wireless apparatus of the radiation imaging system more easily and more accurately.

Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to embodiments, it is to be understood that the invention is not limited to the disclosed embodiments. The scope of the following claims is to be accorded the broadest interpretation so as to encompass all such modifications and equivalent structures and functions.

**Claims**

1. A radiation imaging system (100) comprising:

    a communication device (104) configured to perform wireless communication with a radiation imaging apparatus (101) configured to capture a radiographic image; and
    an information processing apparatus (102),

wherein the information processing apparatus (102) is configured to cause the communication device (104) to perform the wireless communication with the radiation imaging apparatus (101), based on information about a distance between the communication device (104) and the radiation imaging apparatus (101) and a threshold value set in advance.

2. The radiation imaging system (100) according to claim 1, wherein the information processing apparatus (102) is configured to cause the communication device to perform the wireless communication with the radiation imaging apparatus (101) in a case where the information about the distance exceeds the threshold value, and not cause the communication device (104) to perform the wireless communication with the radiation imaging apparatus (101) in a case where the information about the distance does not exceed the threshold value.

3. The radiation imaging system (100) according to claim 1 or 2, wherein the radiation imaging apparatus (101) includes first wireless communication means (2) for performing wireless communication, different from the wireless communication, with an access point included in the radiation imaging system (103) in order to control the capturing of the image, and second wireless communication means (6) for performing the wireless communication in order to make a setting to be used for the different wireless communication.

4. The radiation imaging system (100) according to claim 3, wherein the first wireless communication means (2) performs the different wireless communication with the access point via a wireless local area network (WLAN), and the second wireless communication means (6) performs the wireless communication with the communication device via a wireless personal area network (WPAN).

5. The radiation imaging system (100) according to claim 4, wherein the communication device (104) and the second wireless communication means (6) support at least one of a Bluetooth@ Basic Rate/Enhanced Data Rate standard and a Bluetooth@ Low Energy standard.

6. The radiation imaging system (100) according to any one of claims 1 to 5, wherein the information about the distance is based on signal strength of a signal from the communication device (104) acquired by the radiation imaging apparatus (101).

7. The radiation imaging system (100) according to claim 6, wherein the information about the distance is the signal strength corresponding to the distance between the communication device (104) and the radiation imaging apparatus (101).

8. The radiation imaging system (100) according to claim 7, wherein, in a case where the signal strength exceeds the threshold value, the information processing apparatus (102) causes the communication device (104) to perform the wireless communication with the radiation imaging apparatus (101).

9. The radiation imaging system (100) according to any one of claims 6 to 8, wherein the signal strength is acquired by the second wireless communication means (6).

10. The radiation imaging system (100) according to claim 8, wherein the threshold value is set with respect to the signal strength in a case where the distance is one meter or less.

11. The radiation imaging system (100) according to any one of claims 1 to 10,

wherein the information processing apparatus (102) executes at least one of a plurality of modes for making a response in a case where the communication device (104) is requested by the radiation imaging apparatus (101) to perform the wireless communication,
wherein, in an automatic mode among the plurality of modes, a response is made based on the distance and the threshold value,
wherein, in a manual mode among the plurality of modes, a response is made based on an input using input means included in the information processing apparatus, and
wherein, in a semi-manual mode among the plurality of modes, a response is made based on the response in the automatic mode and the response in the manual mode.

12. The radiation imaging system (100) according to claim 11, wherein, in the semi-manual mode, the response is made based on at least one of a logical sum and a logical product of the response in the automatic mode and the response in the manual mode.

13. The radiation imaging system (100) according to any one of claims 1 to 12, wherein the information processing apparatus (102) is engaged with and connected to the communication device (104).

14. The radiation imaging system (100) according to any one of claims 1 to 12, further comprising:

a plurality of communication devices (104) each configured to perform the wireless communication with the radiation imaging apparatus (101);

and
a plurality of information processing apparatuses (102) each engaged with and connected to a corresponding one of the plurality of communication devices (104).

15. A radiation imaging apparatus (101) configured to capture a radiographic image, the radiation imaging apparatus (101) comprising:

wireless communication means (2, 6) for performing wireless communication with a communication device (104) included in a radiation imaging system (100),
wherein the wireless communication means (2, 6) starts the wireless communication based on information about a distance between the radiation imaging apparatus (101) and the communication device (104) and a threshold value set in advance in the radiation imaging system (100).

16. A control apparatus (102, 105) configured to control a radiation imaging apparatus (101) configured to capture a radiographic image,
wherein, based on information about a distance between a communication device (104) configured to perform wireless communication with the radiation imaging apparatus (101) and the radiation imaging apparatus (101) and a threshold value set in advance, the control apparatus is configured to cause the communication device (104) to perform the wireless communication with the radiation imaging apparatus (101).

17. A method for controlling a radiation imaging system (100) including a communication device (104) configured to perform wireless communication with a radiation imaging apparatus (101) configured to capture a radiographic image, and an information processing apparatus (102), the method comprising:

comparing information about a distance between the communication device (104) and the radiation imaging apparatus (101) with a threshold value set in advance; and
performing the wireless communication based on a result of the comparing.

18. A program that when executed on a computer causes the computer to perform the method according to claim 17.

# FIG.1

100

106 RADIATION GENERATION APPARATUS

105 SYNCHRONIZATION CONTROL APPARATUS

108 IN-HOSPITAL LAN

103 ACCESS POINT

104 COMMUNICATION DEVICE

INFORMATION PROCESSING APPARATUS

102

107

H

101 RADIATION IMAGING APPARATUS

EP 4 111 979 A1

# FIG.2

EP 4 111 979 A1

# FIG.3

```
                    ┌─────────────────────────────┐  S301
              ┌────►│     START COMMUNICATION      │
              │     └─────────────────────────────┘
              │                    │
              │                    ▼              S302
              │         ◄─────────────────────►
        NO ◄──┤         IDENTIFIER MATCHED?
              │         ◄─────────────────────►
              │              YES  │
              │                   ▼             S303
              │         ◄─────────────────────►
        NO ◄──┤          SIGNAL STRENGTH >
              │          THRESHOLD VALUE?
              │         ◄─────────────────────►
              │              YES  │
              │                   ▼
              │     ┌─────────────────────────────┐  S304
              │  ┌─►│      START CONNECTION        │
              │  │  └─────────────────────────────┘
              │  │                │
              │  │                ▼             S305
              │  │      ◄─────────────────────►
         NO ──┤  │      CONNECTION ESTABLISHED?
              │  │      ◄─────────────────────►
              │  │           YES  │
              │  │                ▼
              │  │  ┌─────────────────────────────┐  S306
              │  │ ►│   START GATT COMMUNICATION   │
              │  │  └─────────────────────────────┘
              │  │                │
              │  │                ▼             S307
              │  │      ◄─────────────────────►
         NO ──┘  │      COMMUNICATION COMPLETED?
                 │      ◄─────────────────────►
                 │           YES  │
                 │                ▼             S308
                 │      ◄─────────────────────►
         NO ─────┤       ANY DIFFERENCE
                 │      BETWEEN NEW SETTING
                 │       AND CURRENT SETTING?
                 │      ◄─────────────────────►
                 │           YES  │
                 │                ▼
                 │  ┌─────────────────────────────┐  S309
                 │  │      START CONNECTION        │
                 │  └─────────────────────────────┘
                 │                │
                 │                ▼             S310
                 │  ┌─────────────────────────────┐
                 │  │   STORE SETTING INFORMATION  │
                 │  └─────────────────────────────┘
                 │                │
                 │                ▼
                 └──────────►(     END     )
```

# FIG.4

# FIG.5

| RADIATION IMAGING ROOM 1 | RADIATION IMAGING ROOM 2 |

AREA BASED ON
THRESHOLD VALUE SET
FOR COMMUNICATION
DEVICE 104-1

AREA BASED ON
THRESHOLD VALUE SET
FOR COMMUNICATION
DEVICE 104-2

*102-1*
INFORMATION
PROCESSING
APPARATUS

*101*
RADIATION
IMAGING
APPARATUS

*104-2*
COMMUNICATION
DEVICE

*104-1*
COMMUNICATION
DEVICE

*102-2*
INFORMATION
PROCESSING
APPARATUS

# FIG.6

FIRST WIRELESS COMMUNICATION UNIT — 2

SECOND WIRELESS COMMUNICATION UNIT — 6

COMMUNICATION DEVICE — 104

ACCESS POINT — 103

S601 TRANSMIT ADVERTISING PACKET (DEVICE IDENTIFIER)

S602 TRANSMIT SCAN_REQ

S603 TRANSMIT SCAN_RSP

S604 REQUEST CONNECTION

S605 ESTABLISH CONNECTION

S606 TRANSMIT SSID of "X"

S607 TRANSMIT KEY of "ABCDEFGH"

S608 DISCONNECT

ESTABLISH CONNECTION WITH SSID of "X" AND KEY of "ABCDEFGH"

S609

Europäisches
Patentamt

European
Patent Office

Office européen
des brevets

# EUROPEAN SEARCH REPORT

Application Number

EP 22 18 1396

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/104131 A1 (VAREX IMAGING CORPORTION [US]) 31 May 2019 (2019-05-31) * paragraph [0015] - paragraph [0057] * ----- | 1-18 | INV. A61B6/00 |
| X | EP 3 132 746 A1 (CANON KK [JP]) 22 February 2017 (2017-02-22) * paragraph [0014] - paragraph [0236] * ----- | 1-18 | |
| A,D | EP 2 322 086 A1 (CANON KK [JP]) 18 May 2011 (2011-05-18) * paragraph [0027] - paragraph [0062] * ----- | 1-18 | |

**TECHNICAL FIELDS
SEARCHED      (IPC)**

A61B
H04W

The present search report has been drawn up for all claims

| Place of search | Date of completion of the search | Examiner |
|---|---|---|
| Munich | 16 November 2022 | Hooper, Martin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.......................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 18 1396

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2022

| Patent document cited in search report | | Publication date | Patent family member(s) | | Publication date |
|---|---|---|---|---|---|
| WO 2019104131 | A1 | 31-05-2019 | US | 2019166585 A1 | 30-05-2019 |
| | | | WO | 2019104131 A1 | 31-05-2019 |
| EP 3132746 | A1 | 22-02-2017 | CN | 106388840 A | 15-02-2017 |
| | | | EP | 3132746 A1 | 22-02-2017 |
| | | | EP | 3656306 A2 | 27-05-2020 |
| | | | JP | 6585957 B2 | 02-10-2019 |
| | | | JP | 2017029410 A | 09-02-2017 |
| | | | KR | 20170015217 A | 08-02-2017 |
| | | | US | 2017031035 A1 | 02-02-2017 |
| EP 2322086 | A1 | 18-05-2011 | CN | 102076119 A | 25-05-2011 |
| | | | CN | 103735281 A | 23-04-2014 |
| | | | CN | 103750849 A | 30-04-2014 |
| | | | EP | 2322086 A1 | 18-05-2011 |
| | | | JP | 5680935 B2 | 04-03-2015 |
| | | | JP | 2011120885 A | 23-06-2011 |
| | | | KR | 20110053196 A | 19-05-2011 |
| | | | KR | 20140058472 A | 14-05-2014 |
| | | | US | 2011116486 A1 | 19-05-2011 |
| | | | US | 2014177806 A1 | 26-06-2014 |

EPO FORM P0459

For more details about this annex : see Official Journal of the European Patent Office, No. 12/82

**REFERENCES CITED IN THE DESCRIPTION**

**Patent documents cited in the description**

- JP 2011120885 A